(19) Europäisches Patentamt
European Patent Office
Office européen des brevets

(11) **EP 2 892 960 B1**

(12) **EUROPÄISCHE PATENTSCHRIFT**

(45) Veröffentlichungstag und Bekanntmachung des Hinweises auf die Patenterteilung:
**28.08.2024   Patentblatt 2024/35**

(21) Anmeldenummer: **13752587.9**

(22) Anmeldetag: **16.08.2013**

(51) Internationale Patentklassifikation (IPC):
*C09C 1/00* (2006.01)      *A61K 8/29* (2006.01)
*A61Q 1/00* (2006.01)      *A61Q 1/02* (2006.01)
*C09D 5/36* (2006.01)      *A61K 8/19* (2006.01)

(52) Gemeinsame Patentklassifikation (CPC):
**C09C 1/0015; A61K 8/19; A61K 8/29; A61Q 1/00; A61Q 1/02; C09C 1/0021; C09C 1/0039; C09D 5/36;** A61K 2800/436; C01P 2004/54; C01P 2004/61; C01P 2006/62; C01P 2006/63; C01P 2006/64; C01P 2006/65;           (Forts.)

(86) Internationale Anmeldenummer:
**PCT/EP2013/002471**

(87) Internationale Veröffentlichungsnummer:
**WO 2014/037079 (13.03.2014 Gazette 2014/11)**

(54) **GOLDPIGMENT**

GOLD PIGMENT

PIGMENT D'OR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.09.2012   DE 102012017608**

(43) Veröffentlichungstag der Anmeldung:
**15.07.2015   Patentblatt 2015/29**

(73) Patentinhaber: **Merck Patent GmbH**
**64293 Darmstadt (DE)**

(72) Erfinder:
• **HONEIT, Ute**
**64289 Darmstadt (DE)**
• **KLEIN, Sylke**
**64380 Rossdorf (DE)**

(74) Vertreter: **Merck Patent Association**
**Merck Patent GmbH**
**64271 Darmstadt (DE)**

(56) Entgegenhaltungen:
EP-A1- 0 307 747      EP-A1- 1 029 900
EP-A1- 1 980 594      EP-A2- 0 763 573
WO-A1-01/30921

(52) Gemeinsame Patentklassifikation (CPC): (Forts.)
C01P 2006/66; C09C 2200/1004; C09C 2200/102;
C09C 2200/301; C09C 2200/302

**Beschreibung**

Die vorliegende Erfindung betrifft goldene Interferenzpigmente, insbesondere für Druckverfahren geeignete goldene Interferenzpigmente, die auf einem spezifischen Substrat mindestens eine Schicht aufweisen, die $Fe_2O_3$ und $TiO_2$ enthält, ein Verfahren zu deren Herstellung sowie deren Verwendung.

Goldene beziehungsweise goldfarbene Gegenstände sind seit jeher mit der Anmutung von Schönheit, Wertigkeit und Exklusivität verbunden. Dabei besteht der wesentliche Unterschied zwischen den Farbstellungen "Gelb" und "Gold" im Prinzip lediglich aus dem, dem Goldton noch zusätzlich zuordenbaren, Glanz, der letztendlich Aufmerksamkeit und Begehren auf sich zieht.

Produkte, die Exklusivität und Luxus vermitteln sollen, werden daher seit langer Zeit mit Verpackungen oder Dekorationen versehen, die mehr oder weniger das Aussehen von purem Gold imitieren. Neben den vielen Anforderungen, die in der heutigen Zeit an moderne Verpackungen und dergleichen geknüpft sind, beispielsweise Schadstoffarmut oder -freiheit, gute Recyclingmöglichkeiten und/oder günstige Rohstoffe, sind insbesondere auch einfache, schnelle und preisgünstige Herstellungsverfahren für die Erzeugung der Endprodukte, beispielsweise Verpackungsmaterialien, zunehmend gefragt. Beschichtungsverfahren, insbesondere Druckverfahren, bieten sich daher zur Erzeugung von Verpackungs-, Dekorations- oder auch Sicherheitsprodukten an.

Goldfarbene Dekore werden daher immer häufiger mittels Beschichtungs- und Druckverfahren auf die jeweiligen Substrate aufgebracht. Jedes der Beschichtungsverfahren erfordert jedoch, technologisch bedingt, unterschiedliche Eigenschaften der jeweils enthaltenen Pigmente, die das optische Erscheinungsbild prägen. Glanzeffekte, die einem Goldglanz ähnlich sind, können mit reinen Absorptionspigmenten nicht erhalten werden, so dass hierfür zumeist die so genannten Effektpigmente eingesetzt werden. Diese bestehen, sofern es sich um Interferenzpigmente handelt, in der Regel aus transparenten plättchenförmigen Trägermaterialien, die mit einer oder mehreren dünnen Schichten aus verschiedenen Materialien, zumeist Metalloxiden, belegt sind, wobei das optische Zusammenspiel von Träger und Beschichtung zu Interferenzeffekten führt und neben Interferenzfarben auch Glanz erzeugt wird.

Es hat sich aber herausgestellt, dass plättchenförmige Effektpigmente im Anwendungsmedium abhängig von ihrer Teilchengröße entweder vermehrt Glanz- und Glitzereffekte erzeugen, was bei gröberen Teilchengrößen der Fall ist, oder aber, bei kleineren Teilchengrößen, auch bei an sich im Wesentlichen transparenten Effektpigmenten ein vergleichsweise gutes Deckvermögen erzielbar ist. Diese Effekte sind aber gegenläufig, so dass beim Einsatz von Interferenzpigmenten stets Kompromisse zwischen gewünschtem Glanz und ebenso gewünschtem hohen Deckvermögen eingegangen werden müssen.

Gleichzeitig lassen die verschiedenen Druckverfahren ebenfalls nur bestimmte Teilchengrößen von eingesetzten Feststoffen zu, so dass insbesondere bei Druckverfahren, die nur mit sehr feinteiligen Pigmenten durchgeführt werden können, beispielsweise das Offsetdruckverfahren und das Intagliodruckverfahren, kaum eine Möglichkeit besteht, mit herkömmlichen feinteiligen Effektpigmenten überhaupt einen nennenswerten Glanz erzielen zu können. Außerdem sind gerade beim Offsetverfahren die erzielbaren Schichtdicken sehr dünn, was wiederum die mit Effektpigmenten erzielbaren Farbeffekte, insbesondere die Farbsättigung, verringert.

Darüber hinaus werden vom menschlichen Auge kalte, grünliche Gelbtöne nicht als "richtiges" Gelb oder Gold wahrgenommen. Dies ist eher bei warmen, leicht rötlichen Gelbtönen der Fall. Außerdem zeigen die üblichen Goldeffektpigmente oft sehr hohe Helligkeitswerte, so dass damit erzeugte Druckbilder im Glanzwinkel zwar gleißend hell, aber nicht in einem satten Goldton erscheinen.

Goldfarbige Effektpigmente, die auf Interferenzerscheinungen basieren, sind in einiger Vielfalt bekannt. Sie basieren in der Regel auf natürlichem oder synthetischem Glimmer, Glasplättchen oder $SiO_2$-Plättchen und weisen Interferenzschichten auf, die unter anderem aus $TiO_2$, aus $Fe_2O_3$ oder aus Gemischen beider Metalloxide bestehen können.

So sind beispielsweise aus DE 196 18 569 A1 mehrschichtige Interferenzpigmente bekannt, die auf einem transparenten Trägermaterial ein Schichtsystem aus abwechselnd hoch- und niedrig brechenden Materialien aufweisen. Sofern die hochbrechenden Schichten $Fe_2O_3$ enthalten, werden Pigmente mit orangeroter Körperfarbe und gegebenenfalls Interferenzfarben im ähnlichen Farbspektrum erhalten.

In DE 199 15 153 A1, DE 199 51 871 A1 und DE 199 51 869 A1 werden jeweils farbstarke Interferenzpigmente beschrieben, die auf einem transparenten Trägermaterial Mehrschichtsysteme aufweisen, wobei mindestens eine der Schichten ein Gemisch aus $TiO_2$ und $Fe_2O_3$ oder Pseudobrookit enthält. Die erhaltenen Glanzpigmente weisen Körperfarben im orangeroten Bereich und/oder eine meist grünstichige goldene Interferenz auf. Die Beispiele zeigen Pigmente auf der Basis von Glimmer mit einer Partikelgröße von 10-60 $\mu$m.

Aus EP 1 980 594 A1 sind Effektpigmente auf der Basis von Glassubstraten mit bestimmter Zusammensetzung bekannt. Der $SiO_2$-Anteil in den Glassubstraten beträgt 65-75 Gew.-%. Diese Glassubstrate können mit verschiedenen Metalloxiden beschichtet sein, beispielsweise mit Gemischen aus $TiO_2$ und $Fe_2O_3$ oder mit $TiFe_2O_5$. Die Glassubstrate weisen eine Dicke im Bereich von 0,05 bis 10 $\mu$m und eine mittlere Partikelgröße im Bereich von 1 bis 1000 $\mu$m auf.

EP 0 307 747 A1 beschreibt goldfarbene Farbglanzpigmente auf der Basis von Glimmer, die eine Schicht aus vorzugsweise Pseudobrookit aufweisen. Die Pigmente haben einen Teilchendurchmesser im Bereich von 2 bis 1000 $\mu$m und eine Dicke im Bereich von 0,05 bis 1 $\mu$m.

In EP 0 763 573 A2 sind plättchenförmige Aluminiumoxidpigmente beschrieben, die Titanoxid enthalten, ein Verfahren zu deren Herstellung sowie Perlglanzpigmente, die solche Aluminiumoxidpigmente als Substrate enthalten. Teilchengrößen im Bereich von 5-60 $\mu$m sind beschrieben.

Die im Stand der Technik beschriebenen, auf Glimmer basierenden Pigmente sind schon auf Grund ihrer Partikelgrößen nicht oder nicht gut für spezifische Druckverfahren wie z.B. Offsetdruck oder Intagliodruck geeignet, weil sie die Druckformen ungenügend füllen bzw. verstopfen oder sich im Applikationsmedium nicht richtig ausrichten können. Werden sie in geringeren Partikelgrößen in solchen Druckverfahren eingesetzt, reicht dagegen das erzielbare Deckvermögen und insbesondere der Glanz nicht aus, um den erwünschten satten, glänzenden Goldton zu erzeugen. Dieses Ziel kann auch mit den überwiegend grünstichigen Goldtönen nicht erreicht werden.

Aufgabe der vorliegenden Erfindung ist es daher, ein goldenes Interferenzpigment zur Verfügung zu stellen, welches auf einem transparenten Trägermaterial eine zur Interferenz fähige Beschichtung aufweist und sowohl in der Körperfarbe als auch in der Interferenzfarbe einen warmen, vom Blickwinkel unabhängigen, kräftigen rötlichen Goldton, einen hohen Glanz sowie ein hohes Deckvermögen zeigt, für nahezu alle gängigen Druckverfahren geeignet ist und im Anwendungsmedium zu satten, warmen, glänzenden Goldtönen bei den erhaltenen Druckbildern führt.

Aufgabe der vorliegenden Erfindung ist es weiterhin, ein Verfahren zur Herstellung eines derartigen goldenen Interferenzpigmentes zur Verfügung zu stellen.

Eine weitere Aufgabe der Erfindung besteht darin, die Verwendung der erfindungsgemäßen Interferenzpigmente aufzuzeigen.

Die Aufgabe der vorliegenden Erfindung wird gelöst durch ein goldenes Interferenzpigment, welches ein plättchenförmiges Substrat und mindestens eine auf dem Substrat befindliche Schicht umfasst, wobei es sich bei dem plättchenförmigen Substrat um ein transparentes, synthetisch erzeugtes Substrat handelt, welches als solches eine grüne Eigeninterferenzfarbe aufweist, wobei sich auf dem Substrat mindestens eine Schicht befindet, die eine Mischung oder ein Mischoxid aus $Fe_2O_3$ und $TiO_2$ enthält, gemäß Anspruch 1.

Des Weiteren wird die Aufgabe der vorliegenden Erfindung durch ein Verfahren zur Herstellung des erfindungsgemäßen goldenen Interferenzpigmentes gemäß Anspruch 7 gelöst, wobei ein synthetisch erzeugtes transparentes Substrat, welches eine grüne Eigeninterferenzfarbe aufweist, mit mindestens einer Schicht belegt wird, die eine Mischung oder ein Mischoxid aus $Fe_2O_3$ und $TiO_2$ enthält.

Die Aufgabe der Erfindung wird ebenfalls durch die Verwendung des erfindungsgemäßen goldenen Interferenzpigmentes in Farben, Lacken, Druckfarben, Kunststoffen, Gläsern, Papier, Keramik, kosmetischen Formulierungen, zur Lasermarkierung von Kunststoffen oder Papier sowie zur Herstellung von Pigmentpräparationen und Trockenpräparaten gelöst.

Bei dem Substrat, welches für die Herstellung des erfindungsgemäßen goldenen Interferenzpigmentes verwendet wird, handelt es sich um ein transparentes, synthetisch erzeugtes plättchenförmiges Substrat, welches als solches bereits eine grüne Eigeninterferenzfarbe aufweist.

Als transparent im Sinne der vorliegenden Erfindung gilt ein Substratplättchen dann, wenn es eingestrahltes sichtbares Licht im Wesentlichen, also zu mindestens 80%, transmittiert. Außerdem weisen die erfindungsgemäß eingesetzten Substratplättchen keine Absorptionsfarbe auf.

Bei den erfindungsgemäß eingesetzten Substratplättchen handelt es sich um synthetisch erzeugte plättchenförmige Substrate homogener Zusammensetzung, die eine obere und eine untere Oberfläche aufweisen, welche die Hauptflächen des jeweiligen Plättchens bilden und parallel zueinander angeordnet sind. Dabei bedeutet parallel im Sinne der vorliegenden Erfindung nicht nur parallel im geometrischen Sinne, sondern schließt Abweichungen der Stellung der Oberflächen zueinander im Vergleich zur geometrischen Parallelität von bis zu 15° ein. Die Länge bzw. Breite dieser Hauptflächen des jeweiligen Substratplättchens stellt in ihrer jeweils längsten Abmessung die Partikelgröße des Substratplättchens dar, während der mittlere Abstand zwischen den Substratoberflächen die geometrische Dicke des jeweiligen Substratplättchens und die gemittelte Dicke aller Substratplättchen die geometrische Dicke der Substrate darstellt.

Des weiteren weisen die erfindungsgemäß eingesetzten synthetisch erzeugten plättchenförmigen Substrate ebene und sehr glatte Oberflächen auf. Durch die synthetische Erzeugung der Substratplättchen lassen sich die Oberflächeneigenschaften, die geometrische Dicke, die Partikelgröße und im besten Falle auch die Partikelgrößenverteilung mittels der Prozessparameter bei der Herstellung der Substratplättchen präzise kontrollieren und einstellen, was bei natürlichen Materialien wie beispielsweise Glimmer, Talk oder Kaolin, die üblicherweise ebenso als Substratmaterialien für Interferenzpigmente Verwendung finden, nicht gewährleistet werden kann.

Bedingt durch die sehr ebenen, parallelen Oberflächen der Substratplättchen, ihre homogene Zusammensetzung und das Fehlen einer Absorptionsfarbe reflektieren die Substratplättchen in einem sie umgebenden klaren, transparenten Medium mit von den Plättchen verschiedener Brechzahl mindestens 5% und bis zu 20 %, insbesondere 6 bis 20%, des eingestrahlten sichtbaren Lichtes, abhängig von der jeweiligen Brechzahl der Plättchen. Dabei ist der reflektierte Lichtanteil umso größer, je größer die Brechzahl des jeweils eingesetzten Plättchenmaterials ist. Diese Reflexion an den jeweiligen Grenzflächen zum Umgebungsmedium führt in Verbindung mit dem entstehenden Gangunterschied zur Interferenz der reflektierten Lichtstrahlen und damit zu einer Eigeninterferenzfarbe der Substratplättchen.

Die erfindungsgemäß eingesetzten Substratplättchen weisen eine grüne Eigeninterferenzfarbe (Licht im Wellenlängenbereich von 490 bis 550 nm) auf, welche auf der Basis der diffusen Reflexion oder der Gesamtreflexion der Substratplättchen in einem transparenten, farblosen Medium ermittelt wird.

Zur Bestimmung dieser Eigeninterferenzfarbe wird anhand der mit Hilfe einer entsprechenden Ulbricht-Kugel bestimmten diffusen Reflexion oder der Gesamtreflexion von eingestrahltem sichtbaren Licht (Probe: 10 $\mu$m dicke Beschichtung auf transparenter PET-Folie, enthaltend einen handelsüblichen transparenten, farblosen Tiefdruckbinder sowie 10 Gew.% Substratplättchen) ein L,a,b-Diagramm nach Hunter ermittelt. Die Reflexionswerte für die erfindungsgemäßen Substratplättchen liegen im L,a,b-Diagramm nach Hunter dabei jeweils im Bereich von L > 30, insbesondere von L = 40 bis 80, b= -20 bis +20, insbesondere -10 bis +10, und a <0, insbesondere a= -0,1 bis -20, besonders bevorzugt -0,1 bis -10.

Übliche Pigmentsubstrate weisen entweder keine oder keine überwiegend sicht- und messbare monochrome Interferenzfarbe auf. So sind Glimmerplättchen, unabhängig davon, ob sie auf natürlichem oder synthetisch erzeugtem Glimmer beruhen, wegen ihres schichtweisen Aufbaus von Silikatschichten und der damit nicht planaren Oberflächen nicht als solche zu einer derartigen Interferenz fähig, die sich als einheitlich wahrnehmbare, überwiegende, monochrome Interferenzfarbe manifestiert. Vielmehr schimmern Glimmerplättchen bei größerer Schichtdicke abhängig vom Betrachtungswinkel in verschiedenen Farben, was bei einer Schüttung aus puren Glimmerplättchen zu einem weißlichen, undefinierten Gesamtfarbeindruck führt.

Unter der Voraussetzung, dass die Substratplättchen ebene und parallele Substratoberflächen aufweisen, werden die optischen Eigenschaften der erfindungsgemäß eingesetzten Substrate im Wesentlichen von der Brechzahl des Substratmaterials sowie von der geometrischen Dicke des Substrates bestimmt.

Dabei kann die Brechzahl des Substratmaterials, bedingt durch den Gehalt an ggf. vorhandenen Fremdoxiden, aber auch durch eingeschlossene Poren oder abhängig von der Kristallmodifikation der vorzugsweise eingesetzten Metalloxide teilweise von der idealen Brechzahl der reinen Substratmaterialien (Bulkmaterial, gemessen unter Standardbedingungen, z. B. nach Landolt-Börnstein) abweichen, so dass die geometrische Schichtdicke der Substrate je nach Herstellungsbedingungen und Materialeinsatz entsprechend angepasst werden muss, um die gewünschte Interferenzfarbe zu erzielen.

Um für die Pigmentherstellung geeignete Substratdicken erhalten zu können, sollte die Brechzahl n des Substratmaterials mindestens größer als 1,5 sein und bevorzugt mindestens 1,65 betragen. Als Material für das Substrat kommen daher dielektrische Materialien oder Materialgemische in Frage, bei denen das Material oder Materialgemisch jeweils eine Brechzahl n von größer als 1,5, bevorzugt von mindestens 1,65 aufweist.

Dabei handelt es sich um farblose Materialien oder Materialgemische.

Des Weiteren ist es erforderlich, dass das Substrat des erfindungsgemä-ßen goldenen Interferenzpigmentes eine Brechzahl $n_1$ aufweist, die zu der Brechzahl $n_2$ einer auf dem Substrat aufzubringenden Interferenzschicht einen Abstand $\Delta$n von mindestens 0,1, besser von mindestens 0,2 aufweist.

Als Materialien für das Substrat des erfindungsgemäßen Interferenzpigmentes sind daher die nachfolgenden farblosen Metalloxide oder speziellen Glasmaterialien mit einer Brechzahl n im Bereich von > 1,5 bis 2,5, insbesondere von 1,65 bis 2,5, geeignet, nämlich Substratplättchen, die aus $Al_2O_3$, aus $Al_2O_3$ mit einem Gehalt von bis zu 5 Gew.% $TiO_2$, bezogen auf das Gewicht des Substrates, aus $ZrO_2$ oder aus $TiO_2$ bestehen, oder solche Substratplättchen, die $Al_2O_3$, $ZrO_2$ oder $TiO_2$ mit einem Anteil von mindestens 90 Gew.%, bezogen auf das Gewicht des Substrates, enthalten. Dabei kann $TiO_2$ in der Anatas- oder der Rutilmodifikation vorliegen.

Weitere Bestandteile der transparenten Substratplättchen können die Oxide oder Oxidhydrate von Sn, Si, Ce, Al, Ca, Zn und/oder Mg sein, die jedoch höchstens mit einem Anteil von 10 Gew.%, bezogen auf das Gewicht des Substrates, in diesem vorliegen und die optischen Eigenschaften, insbesondere die Interferenzfarbe, des Substrates nicht wesentlich bestimmen.

Als Substratmaterial geeignet sind auch Glasplättchen, die die Anforderungen an die Brechzahl erfüllen. Das ist insbesondere bei Plättchen aus Glasmaterial der Fall, deren Anteil an $SiO_2$ höchstens 70 Gew.% beträgt. Darüber hinaus enthalten solche Glasmaterialien noch Anteile an $Al_2O_3$, CaO, MgO, $B_2O_3$, $Na_2O$, $K_2O$, $TiO_2$, ZnO, BaO, $Li_2O$, $ZrO_2$, $Nb_2O_5$, $P_2O_5$ und/oder PbO in wechselnder Zusammensetzung und wechselnden Mengenanteilen. Besonders bevorzugt sind hochbrechende Glasmaterialien wie Flintglas und Schwerflintglas.

Abhängig vom eingesetzten Material weisen die erfindungsgemäß geeigneten Substratplättchen eine geometrische Dicke im Bereich von 100 bis 600 nm auf, wie nachfolgend im Detail ausgeführt.

Voraussetzung für die Eignung als Pigmentsubstrat ist außerdem, dass sich die Substrate auf synthetischem Wege in der jeweils gewünschten Schichtdicke als planare Plättchen herstellen lassen, was bei den angegebenen Materialien jedoch der Fall ist. Außerdem ist es äußerst vorteilhaft, wenn die erfindungsgemäß eingesetzten Pigmentsubstrate in kristalliner Form und insbesondere bevorzugt in Form von Einkristallen vorliegen.

Um eine grüne Eigeninterferenz der Substratplättchen erhalten zu können, weisen Substratplättchen aus $Al_2O_3$ oder aus $Al_2O_3$ mit einem Gehalt von bis zu 5 Gew.% $TiO_2$, bezogen auf das Gewicht des Substrates, sowie solche Substratplättchen, die $Al_2O_3$ mit einem Anteil von mindestens 90 Gew.%, bezogen auf das Gewicht des Substrates, enthalten, eine geometrische Dicke im Bereich von 180 bis 250 nm oder von 350 bis 450 nm auf.

Substratplättchen aus $TiO_2$ oder solche, die $TiO_2$ mit einem Anteil von mindestens 90 Gew.% enthalten, bezogen auf das Gewicht des Substrates, weisen erfindungsgemäß eine geometrische Dicke im Bereich von 110 bis 170 nm oder im Bereich von 240 bis 310 nm auf.

Für Substratplättchen, die aus $ZrO_2$ bestehen, oder solche Substratplättchen, die $ZrO_2$ mit einem Anteil von mindestens 90 Gew.%, bezogen auf das Gewicht des Substrates, enthalten, liegt die geometrische Dicke des Substrates erfindungsgemäß zwischen 140 und 210 nm oder im Bereich von 260 bis 400 nm.

Glasplättchen, die maximal 70 Gew.% $SiO_2$ enthalten, weisen eine geometrische Dicke von 230 bis 300 nm oder von 400 bis 470 nm auf.

Besonders bevorzugt werden als Substrate Plättchen aus $Al_2O_3$ oder aus $Al_2O_3$ mit einem Gehalt von bis zu 5 Gew.% $TiO_2$, bezogen auf das Gewicht des Substrates, die beide im Folgenden zusammenfassend als Aluminiumdioxidplättchen bezeichnet werden, eingesetzt, wobei die Plättchen eine geometrische Dicke im Bereich von 180 bis 250 nm, vorzugsweise im Bereich von 190 bis 230 nm, aufweisen. Diese können, wie später beschrieben wird, in Form von Einkristallen hergestellt werden.

Die Standardabweichung in der Dicke der einzelnen Substratplättchen beträgt dabei vorzugsweise nicht mehr als 10%, bezogen auf den Mittelwert der jeweiligen Substratdicke. Eine solche verhältnismäßig geringe Dickenabweichung kann über das jeweilige Herstellungsverfahren gesteuert werden.

Die Teilchengröße der Substratpartikel ist vergleichsweise gering, da nur feinteilige Partikel in den verschiedensten Druckanwendungen einsetzbar sind. Sie liegt erfindungsgemäß im Bereich von 5-40 $\mu$m. Dabei liegt der $d_{50}$-Wert der Teilchengrößenverteilung erfindungsgemäß im Bereich von 10 bis 25 $\mu$m, vorzugsweise im Bereich von 15 bis 20 $\mu$m, insbesondere im Bereich von 15 $\mu$m bis <20 $\mu$m. Der $d_{95}$-Wert der Teilchengrößenverteilung liegt erfindungsgemäß im Bereich von 35 bis 40 $\mu$m, insbesondere von 35 $\mu$m bis < 40 $\mu$m. Dabei handelt es sich um eine enge Teilchengrößenverteilung, die über die Verfahrensparameter im Herstellungsverfahren und/oder über zusätzliche Mahl- und/oder Klassifizierschritte eingestellt werden kann. Die Teilchengröße und die Teilchengrößenverteilung können über verschiedene fachübliche Methoden ermittelt werden. Bevorzugt wird erfindungsgemäß jedoch die Laserbeugungsmethode in einem Standardverfahren mittels einem Malvern Mastersizer 2000, APA200 (Produkt der Fa. Malvern Instruments Ltd., UK) eingesetzt. Dieses Verfahren hat den Vorteil, dass Partikelgröße und Partikelgrößenverteilung gleichzeitig unter Standardbedingungen bestimmt werden können.

Die Partikelgröße sowie die Dicke von Einzelpartikeln lässt sich außerdem mit Hilfe von SEM (Scanning Electron Microscope) Bildern ermitteln. Bei diesen können Partikelgröße und geometrische Partikeldicke über direktes Ausmessen ermittelt werden. Zur Ermittlung von Durchschnittswerten werden mindestens 1000 Partikel einzeln ausgewertet und die Ergebnisse gemittelt.

Der Formfaktor der Trägerplättchen, also das Verhältnis von Länge bzw. Breite zu Dicke, beträgt im allgemeinen von 2:1 bis 1.000:1, insbesondere von 5:1 bis 500:1 und ganz besonders bevorzugt von 20:1 bis 300:1.

Es ist ein besonderer Vorteil der goldenen Interferenzpigmente gemäß der vorliegenden Erfindung, dass bedingt durch die glatten und planaren Oberflächen der synthetisch erzeugten Substrate und durch die dem Substrat zuzurechnende vergleichsweise hohe Brechzahl auch bei verhältnismäßig geringen Teilchengrößen der Substrate im Anwendungsmedium Glanzwerte erhalten werden können, die mit gleich großen herkömmlichen Interferenzpigmenten, beispielsweise auf Glimmerbasis, nicht erzielbar sind.

Die erfindungsgemäßen goldenen Interferenzpigmente weisen auf dem Substrat, welches als solches eine grüne Interferenzfarbe aufweist, mindestens eine Schicht auf, die eine Mischung oder ein Mischoxid aus $Fe_2O_3$ und $TiO_2$ enthält.

Vorzugsweise besteht diese Schicht entweder aus einer Mischung aus $Fe_2O_3$ und $TiO_2$ oder aus einem Mischoxid derselben, kann diese jedoch auch wahlweise zu mindestens 80 Gew.%, insbesondere zu mindestens 90 Gew. %, bezogen auf das Gewicht des Substrates, sowie zusätzlich 10 bis 20 Gew.% weitere Metalloxide, ausgewählt aus $Al_2O_3$, $Ce_2O_3$, $B_2O_3$, $ZrO_2$ und $SnO_2$, entweder einzeln oder im Gemisch, enthalten. Dabei handelt es sich bei dem Mischoxid vorzugsweise um Pseudobrookit ($Fe_2TiO_5$). Liegt eine Mischung aus $Fe_2O_3$ und $TiO_2$ in der Schicht vor, dann beträgt das molare Verhältnis von $Fe_2O_3$ zu $TiO_2$ von 1:4 bis 4:1, vorzugsweise von 1:2 bis 2:1.

Zusätzlich zu den hohen Brechzahlen der eingesetzten Materialien ($TiO_2$ (Anatas) 2,5, $TiO_2$ (Rutil) 2,7, Hämatit 2,9) weist eine solche Schicht auch eine gelbbraune bis gelbrote Eigenabsorption auf.

Die geometrische Schichtdicke der eine Mischung oder ein Mischoxid aus $Fe_2O_3$ und $TiO_2$ enthaltenden Schicht liegt erfindungsgemäß im Bereich von 20 nm bis 250 nm, insbesondere im Bereich von 50 nm bis 150 nm.

Wie bereits vorab beschrieben beträgt die Brechzahldifferenz $\Delta n$ zwischen Substrat und auf dem Substrat befindlicher Schicht mindestens 0,1, insbesondere mindestens 0,2.

Vorzugsweise befindet sich eine Schicht, die eine Mischung oder ein Mischoxid aus $Fe_2O_3$ und $TiO_2$ enthält,

direkt auf dem Substrat.

Besonders bevorzugt ist jedoch eine Ausführungsform der vorliegenden Erfindung, bei der das goldene Interferenzpigment zwei Schichten aufweist, die eine Mischung oder ein Mischoxid aus $Fe_2O_3$ und $TiO_2$ enthalten.

Im letzteren Falle werden die beiden, jeweils $Fe_2O_3$ und $TiO_2$ enthaltenden Schichten durch mindestens eine weitere Schicht aus einem farblosen, dielektrischen Material voneinander separiert, d.h. die mindestens eine weitere dielektrische Schicht liegt als Zwischenschicht zwischen den beiden jeweils $Fe_2O_3$ und $TiO_2$ enthaltenden Schichten vor.

Ist nur eine einzelne derartige Zwischenschicht vorhanden, handelt es sich erfindungsgemäß um eine Schicht aus einem farblosen dielektrischen Material, welches eine Brechzahl n von $\leq 1,8$ aufweist. Als geeignetes Material hierfür werden $SiO_2$, $Al_2O_3$, deren Oxidhydrate, Mischungen daraus, oder auch $MgF_2$ eingesetzt. Insbesondere bevorzugt besteht die Zwischenschicht aus $SiO_2$, dem entsprechenden Oxidhydrat, oder einer Mischung aus diesen. Die geometrische Schichtdicke dieser Schicht beträgt erfindungsgemäß zwischen 5 und 100 nm, insbesondere zwischen 20 und 50 nm.

Liegt mehr als eine Zwischenschicht vor, wobei die Anzahl der Zwischenschichten vorzugsweise 2 oder 3 ist, so ist als zweite und gegebenenfalls dritte Zwischenschicht zwischen den jeweils eine Mischung oder ein Mischoxid aus $Fe_2O_3$ und $TiO_2$ enthaltenden Schichten noch zusätzlich eine Schicht enthalten, die aus einem farblosen dielektrischen Material, welches eine Brechzahl n von > 1,8 aufweist, besteht. Besonders geeignet hierfür sind farblose Metalloxide wie $TiO_2$ und $ZrO_2$, Oxidhydrate von $TiO_2$ oder $ZrO_2$, oder Mischungen aus den Oxidhydraten und den jeweiligen Oxiden, oder Materialien die $TiO_2$, $ZrO_2$ oder die entsprechenden Oxidhydrate zu einem Anteil von mindestens 80 Gew.%, bezogen auf das Gewicht der Schicht, enthalten und gegebenenfalls bis zu 20 Gew.%, bezogen auf die Schicht, an Fremdoxiden enthalten können. Weitere Bestandteile der hochbrechenden transparenten Schicht können die Oxide oder Oxidhydrate von Sn, Si, Ce, Al, Ca oder Zn sein.

Vorzugsweise wird $TiO_2$ eingesetzt, welches sowohl in der Anatasmodifikation als auch in der Rutilmodifikation vorliegen kann. Die Erzeugung einer Rutilmodifikation ist dem Fachmann geläufig und beispielsweise in den Druckschriften EP 271 767 B1, DE 25 22 572 C2 oder US 6,626,989 beschrieben.

Die geometrische Dicke dieser hochbrechenden Schicht(en) liegt erfindungsgemäß jeweils zwischen 5 und 60 nm, insbesondere im Bereich von 10 bis 60 nm, und besonders bevorzugt im Bereich von 10 bis 50 nm. Die zweite und gegebenenfalls dritte Zwischenschicht befinden sich oberhalb oder unterhalb der Zwischenschicht aus einem farblosen dielektrischen Material, welches eine Brechzahl n von $\leq 1,8$ aufweist, oder beiderseits von letzterer.

Vorzugsweise beträgt die geometrische Dicke der Gesamtheit aller Zwischenschichten jedoch nicht mehr als 100 nm, insbesondere nicht mehr als 50 nm.

In einer besonders bevorzugten Ausführungsform der vorliegenden Erfindung liegt mindestens eine der eine Mischung oder ein Mischoxid aus $Fe_2O_3$ und $TiO_2$ enthaltenden Schichten als Pseudobrookit-Schicht vor. Insbesondere bevorzugt ist eine Ausführungsform der vorliegenden Erfindung, bei der beide Schichten, die eine Mischung oder ein Mischoxid aus $Fe_2O_3$ und $TiO_2$ enthalten, als Pseudobrookit-Schicht vorliegen, sofern zwei dieser Schichten vorhanden sind.

Bei einer weiteren Ausführungsform der vorliegenden Erfindung liegt eine einzelne Schicht vor, die eine Mischung oder ein Mischoxid aus $Fe_2O_3$ und $TiO_2$ enthält, und anstelle einer zweiten Schicht derselben Art ist ein Zweischichtsystem aus einer $Fe_2O_3$-Schicht und einer $TiO_2$-Schicht im erfindungsgemäßen Pigment, inklusive der vorab genannten Zwischenschicht(en), vorhanden. Bedingt durch die dadurch entstehende Vielzahl an Schichten ist jedoch eine solche Ausführungsform nicht besonders bevorzugt.

Sowohl die eine Mischung oder ein Mischoxid aus $Fe_2O_3$ und $TiO_2$ enthaltende(n) Schicht(en) wie auch mindestens eine der ggf. vorhandene(n) Zwischenschicht(en) wirken als optisch aktive Interferenzschichten und tragen daher zur Gesamt-Interferenzfarbe des erfindungsgemäßen Pigmentes bei.

Erfindungsgemäß sind insbesondere die folgenden Schichtsysteme geeignet beziehungsweise bevorzugt. Dabei können die für die jeweiligen Schichten aus Materialien mit einer Brechzahl $\leq 1,8$ bzw. >1,8 hier beispielhaft verwendeten Materialien $SiO_2$ und $TiO_2$ auch durch die oben aufgeführten oder auch weitere geeignete andere Materialien ersetzt werden. Vorzugsweise umhüllen alle genannten Schichten den plättchenförmigen Träger vollständig.

Substrat (G)-$Fe_2O_3$/$TiO_2$
Substrat (G)-$Fe_2TiO_5$
Substrat (G)-$Fe_2O_3$/$TiO_2$-$SiO_2$-$Fe_2O_3$/$TiO_2$
Substrat (G)-$Fe_2O_3$/$TiO_2$-$SiO_2$-$Fe_2TiO_5$
Substrat (G)-$Fe_2TiO_5$-$SiO_2$-$Fe_2O_3$/$TiO_2$
Substrat (G)-$Fe_2O_3$-$TiO_2$-$SiO_2$-$Fe_2O_3$/$TiO_2$
Substrat (G)-$Fe_2O_3$/$TiO_2$-$SiO_2$-$Fe_2O_3$-$TiO_2$
Substrat (G)- $Fe_2O_3$-$TiO_2$-$SiO_2$-$Fe_2TiO_5$
Substrat (G)- $Fe_2TiO_5$-$SiO_2$-$Fe_2O_3$-$TiO_2$
Substrat (G)-$Fe_2TiO_5$-$SiO_2$-$Fe_2TiO_5$

Substrat (G)-$Fe_2O_3$/$TiO_2$-$SiO_2$-$TiO_2$-$Fe_2O_3$/$TiO_2$
Substrat (G)-$Fe_2O_3$/$TiO_2$-$SiO_2$-$TiO_2$-$Fe_2TiO_5$
Substrat (G)-$Fe_2TiO_5$-$SiO_2$-$TiO_2$-$Fe_2O_3$/$TiO_2$
Substrat (G)-$Fe_2O_3$-$TiO_2$-$SiO_2$-$TiO_2$-$Fe_2O_3$/$TiO_2$
Substrat (G)-$Fe_2O_3$/$TiO_2$-$SiO_2$-$TiO_2$-$Fe_2O_3$-$TiO_2$
Substrat (G)- $Fe_2O_3$-$TiO_2$-$SiO_2$-$TiO_2$-$Fe_2TiO_5$
Substrat (G)- $Fe_2TiO_5$-$SiO_2$-$TiO_2$-$Fe_2O_3$-$TiO_2$
Substrat (G)-$Fe_2TiO_5$-$SiO_2$-$TiO_2$-$Fe_2TiO_5$
Substrat (G)-$Fe_2O_3$/$TiO_2$-$TiO_2$-$SiO_2$-$Fe_2O_3$/$TiO_2$
Substrat (G)-$Fe_2O_3$/$TiO_2$-$TiO_2$-$SiO_2$-$Fe_2TiO_5$
Substrat (G)-$Fe_2TiO_5$-$TiO_2$-$SiO_2$-$Fe_2O_3$/$TiO_2$
Substrat (G)-$Fe_2O_3$/$TiO_2$-$TiO_2$-$SiO_2$-$Fe_2O_3$-$TiO_2$
Substrat (G)- $Fe_2TiO_5$-$TiO_2$-$SiO_2$-$Fe_2O_3$-$TiO_2$
Substrat (G)-$Fe_2TiO_5$-$TiO_2$-$SiO_2$-$Fe_2TiO_5$
Substrat (G)-$Fe_2O_3$/$TiO_2$-$TiO_2$-$SiO_2$-$TiO_2$-$Fe_2O_3$/$TiO_2$
Substrat (G)-$Fe_2O_3$/$TiO_2$-$TiO_2$-$SiO_2$-$TiO_2$-$Fe_2TiO_5$
Substrat (G)-$Fe_2TiO_5$-$TiO_2$-$SiO_2$-$TiO_2$-$Fe_2O_3$/$TiO_2$
Substrat (G)-$Fe_2O_3$/$TiO_2$-$TiO_2$-$SiO_2$-$TiO_2$-$Fe_2O_3$-$TiO_2$
Substrat (G)- $Fe_2TiO_5$-$TiO_2$-$SiO_2$-$TiO_2$-$Fe_2O_3$-$TiO_2$
Substrat (G)-$Fe_2TiO_5$-$TiO_2$-$SiO_2$-$TiO_2$-$Fe_2TiO_5$
Insbesondere bevorzugt sind daraus die Schichtsysteme
Substrat (G)-$Fe_2TiO_5$
Substrat (G)-$Fe_2TiO_5$-$SiO_2$-$Fe_2TiO_5$
Substrat (G)-$Fe_2TiO_5$-$SiO_2$-$TiO_2$-$Fe_2TiO_5$
Substrat (G)-$Fe_2TiO_5$-$TiO_2$-$SiO_2$-$Fe_2TiO_5$ sowie
Substrat (G)-$Fe_2TiO_5$-$TiO_2$-$SiO_2$-$TiO_2$-$Fe_2TiO_5$.

Substrat (G) bedeutet jeweils ein transparentes Substratplättchen mit grüner Eigeninterferenz, wie vorab beschrieben. $Fe_2O_3$/$TiO_2$ bedeutet eine Mischung aus $Fe_2O_3$ und $TiO_2$.

Bei den erfindungsgemäßen goldenen Interferenzpigmenten trägt das Substrat mit der ihm eigenen Interferenzfarbe "Grün" in besonders vorteilhafter Weise zu farblichen Gesamteindruck des Pigmentes bei. Die grüne Interferenzfarbe des Substrates führt nämlich gleichzeitig zu einer roten Transmissionsfarbe des Substrates, die auf dem jeweiligen Anwendungsuntergrund, also beispielsweise bei Druckanwendungen auf dem Bedruckstoff, streut. Vor allem bei dickeren Anwendungsschichten, bei denen mehrere Pigmente im Anwendungsmedium übereinander liegen, beispielsweise beim Intagliodruck, verstärkt die so gestreute Transmissionsfarbe "Rot" die rötliche bzw. rötlichgelbe oder braungelbe Absorptionsfarbe der eine Mischung oder ein Mischoxid aus $Fe_2O_3$ und $TiO_2$ enthaltenden Schicht(en) in vorteilhafter Weise hin zum gewünschten Gelb-Rot-Bereich. Gleichzeitig ergibt die Interferenzfarbe "Grün" des Substrates eine Mischfarbe mit der Interferenzfarbe der Beschichtung, also der Interferenzfarbe mindestens der Schicht, die eine Mischung oder ein Mischoxid aus $Fe_2O_3$ und $TiO_2$ enthält, sowie gegebenenfalls der weiteren vorhandenen Interferenzschichten. Wird diese Interferenzfarbe der Beschichtung im Bereich einer roten oder rötlichen Interferenz eingestellt, ergibt sich eine Gesamt-Interferenz im gelbgoldenen Farbbereich. Sowohl die Interferenzfarbe des Substrates als auch die daraus resultierende Transmissionsfarbe des Substrates bewirken damit eine Verstärkung des optischen Gesamteindruckes des erfindungsgemäßen Pigmentes im Rot-Gold-Bereich, und zwar unabhängig vom Betrachtungswinkel. Gleichzeitig weisen die erfindungsgemäβen goldenen Interferenzpigmente, wie oben bereits beschrieben, trotz ihrer vergleichsweise geringen Teilchengrößen im Anwendungsmedium einen starken Glanz auf. Darüber hinaus befinden sich das Deckvermögen sowie die Farbsättigung in einem insbesondere für Druckanwendungen bevorzugt geeigneten Bereich.

Die erfindungsgemäßen goldenen Interferenzpigmente können zusätzlich zu den oben beschriebenen Schichten auf ihrer äußeren Oberfläche noch mit einer anorganischen und/oder organischen, so genannten Nachbeschichtung versehen sein. Diese fachüblich angewendete Nachbeschichtung dient beispielsweise zur Vereinfachung der Einarbeitung in das Anwendungsmedium, zur Verbesserung der Witterungsbeständigkeit, zur Verminderung der Vergilbungsneigung des Anwendungsmediums oder zur besseren Verteilung der Interferenzpigmente im Anwendungsmedium. Nachbeschichtungen verändern die optischen Eigenschaften (Koloristik) der Interferenzpigmente praktisch nicht und sind mit äußerst geringen Schichtdicken, die in der Regel lediglich im Bereich von Molekül-Monolagen bis zu etwa 15 nm, vorzugsweise bis ca. 5 nm, liegen, auf der Pigmentoberfläche vorhanden. Entsprechende Verfahren und Materialien sind dem Fachmann in großer Zahl bekannt und beispielsweise in den Druckschriften DE 22 15 191, DE 31 51 354, DE 32 35 017, DE 33 34 598, EP 0090259, EP 634 459, WO 96/32446, WO 99/57204 und WO 01/92425 beschrieben.

Gegenstand der vorliegenden Erfindung ist auch ein Verfahren zur Herstellung von goldenen Interferenzpigmenten, wobei ein synthetisch erzeugtes transparentes Substrat, welches eine grüne Eigeninterferenzfarbe aufweist, mit mindestens einer Schicht belegt wird, die eine Mischung oder ein Mischoxid aus $Fe_2O_3$ und $TiO_2$ enthält.

Als synthetisch erzeugtes Substrat mit grüner Eigeninterferenzfarbe eignen sich die vorab bereits beschriebenen Substrate, die transparent sind und eine Brechzahl n im Bereich von > 1,5 bis 2,5 und insbesondere von 1,65 bis 2,5 aufweisen, nämlich solche Substratplättchen, die aus $Al_2O_3$, aus $Al_2O_3$ mit einem Gehalt von bis zu 5 Gew.% $TiO_2$, bezogen auf das Gewicht des Substrates, aus $ZrO_2$ oder aus $TiO_2$ bestehen, oder solche Substratplättchen, die $Al_2O_3$, $ZrO_2$ oder $TiO_2$ mit einem Anteil von mindestens 90 Gew.%, bezogen auf das Gewicht des Substrates, enthalten, wie vorab im Detail beschrieben. Weitere Bestandteile der transparenten Substratplättchen können die Oxide oder Oxidhydrate von Sn, Si, Ce, Al, Ca oder Zn sein, die jedoch höchstens mit einem Anteil von 10 Gew.%, bezogen auf das Gewicht des Substrates, in diesem vorliegen.

Auch die vorab bereits beschriebenen Glasplättchen, die bis zu 70 Gew.% an $SiO_2$ und weitere Bestandteile enthalten, sind geeignet.

Je nach Material sind die vorab bereits beschriebenen geometrischen Dicken der Substrate einzuhalten, um eine grüne Eigeninterferenzfarbe der Substratplättchen erhalten zu können.

Die oben beschriebenen, im Wesentlichen aus $Al_2O_3$ bestehenden plättchenförmigen Substrate können dabei vorzugsweise nach dem in der EP 763 573 A2 beschriebenen Verfahren hergestellt werden. Diese Substrate enthalten geringe Mengen an $TiO_2$, was die nachfolgende Beschichtung mit Interferenzschichten erleichtert. Die nach diesem Verfahren hergestellten Aluminiumoxidplättchen werden als Einkristalle in einem Kristallwachstumsprozeß erhalten, bei dem die Teilchengröße der Substrate wie auch deren geometrische Dicke, deren Standardabweichung nicht größer als 10% beträgt, durch die Verfahrensparameter gesteuert werden können. Die entsprechenden Einflussparameter sind dem Fachmann bekannt. Sollen andere Fremdoxide an Stelle von $TiO_2$ enthalten sein, wird analog zum in EP 763 573 A2 beschriebenen Verfahren unter Austausch der Rohstoffe gearbeitet. Es sind jedoch auch Aluminiumdioxidplättchen in der Form hexagonaler Plättchen mit einem Partikeldurchmesser von größer als 10 $\mu$m und einem Aspektverhältnis (Partikeldurchmesser/Dicke) von 5 bis 10, welche aus JP-A 111239/1982 bekannt sind, oder die in JP-A 39362/ 1992 beschriebenen hexagonalen Aluminiumdioxidplättchen geeignet.

Substratplättchen, die ganz oder überwiegend aus $ZrO_2$ , $TiO_2$ deren Oxidhydraten oder Mischungen daraus bestehen, können analog zu dem in WO 93/08237 beschriebenen Verfahren hergestellt werden. Die analog zu diesem Verfahren hergestellten Substratplättchen sollten jedoch keine gelösten oder ungelösten Farbmittel enthalten. Sie werden aus dem entsprechenden, vorzugsweise anorganischen, Precursor-Material in einem Bandverfahren erzeugt, wobei der Precursor auf das Band aufgebracht, mit Säure in die oxidische Form bzw. das Oxidhydrat überführt, verfestigt und anschließend vom Band abgelöst sowie ggf. kalziniert wird. Die geometrische Schichtdicke der Substratplättchen wird über die Auftragsmenge bzw. Nassschichtdicke der Precursorschicht eingestellt, was sehr präzise möglich ist und zu einer engen Dickenverteilung mit Schwankungen von höchstens 10% führt. Die Partikelgröße der Substratplättchen muss über nachfolgende Mahl- und Klassierverfahren eingestellt werden, die jedoch fachüblich sind.

Plättchenförmige Glassubstrate sind in verschiedenen Dicken und Qualitäten von diversen Anbietern am Markt verfügbar, beispielsweise Borosilikat (ECR)-Glasflakes in Dicken von 100 bis 500 nm von der Firma Glassflake Australia Pty Ltd.

Bei dem erfindungsgemäßen Herstellungsverfahren erfolgt die Belegung der plättchenförmigen Substrate mit der nachfolgenden $Fe_2O_3$ und $TiO_2$ enthaltenden Schicht sowie ggf. allen weiteren Interferenzschichten des Schichtsystems vorzugsweise in einer wässrigen Dispersion mittels eines nasschemischen Verfahrens durch hydrolytische Zersetzung von insbesondere anorganischen Metallsalzen.

Die Herstellung von Interferenzpigmenten nach nasschemischen Verfahren aus anorganischen Ausgangsstoffen ist an sich bekannt. Beispielhaft genannt seien hier fachübliche Herstellungsverfahren, wie sie in den Patentschriften DE 14 67 468, DE 19 59 988, DE 20 09 566, DE 22 14, 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602 oder DE 32 35 017 beschrieben sind.

Dazu werden die Substratplättchen in Wasser suspendiert und bei einem für die Hydrolyse geeigneten pH-Wert mit ein oder mehreren hydrolysierbaren Metallsalzen versetzt, so dass die Metalloxidhydrate bzw. Metalloxide auf die Substratplättchen aufgefällt werden. Die Menge der Metallsalze , der pH-Wert sowie die Zugabegeschwindigkeit sollten vorteilhafterweise so ausgewählt werden, dass keine Nebenfällungen entstehen. Der pH-Wert wird im allgemeinen durch die Zugabe einer Säure und/oder Base zeitgleich während der Fällung eingestellt und konstant gehalten. Die erhaltenen Pigmente werden im Anschluss abgetrennt, in der Regel gewaschen, getrocknet und gegebenenfalls geglüht. Die Trocknung erfolgt üblicherweise bei Temperaturen zwischen 50 und 150°C für einen Zeitraum von 6 bis 18 h, während der Glühprozess zeitlich abhängig vom jeweiligen Schichtaufbau erfolgt und in der Regel bei Temperaturen zwischen 250 und 1100°C, vorzugsweise zwischen 350 und 900°C, stattfindet.

Falls erforderlich, wird das geglühte Pigment anschließend gesiebt.

Die Belegung mit verschiedenen Schichten kann jeweils einzeln erfolgen, wobei das Pigment nach jedem Belegungsschritt getrocknet sowie ggf. geglüht und anschließend resuspendiert wird, oder in einem Eintopfverfahren mit nur einem einzigen Wasch-, Trocknungs- und ggf. Glühschritt am Ende der Pigmentherstellung.

Für die Aufbringung der Interferenzschichten eignen sich wasserlösliche anorganische Ausgangsstoffe, respektive Metallsalze, die dem Fachmann seit langem für derartige Verfahren geläufig sind, beispielsweise $FeCl_3$, $Fe(NO_3)_3$, $FeNH_4(SO_4)_2$, $Fe_2(SO_4)_3$ bzw. $TiCl_4$ für die Aufbringung der Schicht, die eine Mischung oder ein Mischoxid aus $Fe_2O_3$ und $TiO_2$ enthält. Diese werden bevorzugt in wässiger Lösung eingesetzt. Besonders bevorzugt werden $FeCl_3$ und $TiCl_4$ eingesetzt.

Um eine Mischung oder ein Mischoxid aus $Fe_2O_3$ und $TiO_2$ direkt auf den Substratplättchen auffällen zu können, ist eine pH-Wert-Einstellung im Bereich von 1,5 bis 4,0 erforderlich, vorzugsweise im Bereich von 2,0 bis 3,0. Die Ausgangsstoffe werden hierfür im jeweils gewünschten Mischungsverhältnis eingesetzt. Ansonsten wird der vorab beschriebenen allgemeinen Verfahrensbeschreibung gefolgt. Die Reaktionstemperatur liegt im Bereich zwischen 50°C und 100°C.

In einer besonders bevorzugten Ausführungsform des erfindungsgemäßen Verfahrens werden auf das plättchenförmige Substrat zwei Schichten aufgebracht, die eine Mischung oder ein Mischoxid aus $Fe_2O_3$ und $TiO_2$ enthalten, wobei zwischen diesen Schichten mindestens eine weitere Schicht aus einem farblosen dielektrischen Material aufgebracht wird, und das Material für diese weitere Schicht eine Brechzahl n von <1,8 aufweist.

Vorzugsweise handelt es sich bei dem Material mit einer Brechzahl n von <1,8 um Siliziumdioxid, Siliziumdioxidhydrat oder eine Mischung daraus. Eine solche Schicht wird nachfolgend als $SiO_2$-Schicht bezeichnet.

Für die Aufbringung einer $SiO_2$-Schicht wird in der Regel eine Natrium- oder Kaliumwasserglaslösung eingesetzt. Die Ausfällung einer Siliziumdioxid- bzw. Siliziumdioxidhydratschicht erfolgt bei einem pH-Wert im Bereich von 6 bis 10, vorzugsweise von 7 bis 9.

Vorzugsweise wird dabei das vorab bereits mit einer Schicht, die eine Mischung oder ein Mischoxid aus $Fe_2O_3$ und $TiO_2$ enthält, beschichtete Substrat in Wasser suspendiert und die Suspension auf eine Temperatur im Bereich von 50 bis 100°C erhitzt. Der pH-Wert wird im Bereich von 6 bis 10 eingestellt und durch gleichzeitige Zugabe einer verdünnten Mineralsäure, beispielsweise von HCl, $HNO_3$ oder $H_2SO_4$, konstant gehalten. Zu dieser Suspension wird eine Natrium- oder Kaliumwasserglaslösung gegeben. Sobald die gewünschte Schichtdicke an $SiO_2$ auf dem beschichteten Substrat erhalten ist, wird die Zugabe der Silikatlösung gestoppt und der Ansatz für weitere 0,5 Stunden nachgerührt.

Alternativ kann eine hydrolytische Beschichtung mit $SiO_2$ auch unter Verwendung organischer Siliziumverbindungen, wie beispielsweise TEOS, in einem sauer oder basisch katalysierten Verfahren über eine Sol-Gel-Reaktion erfolgen. Auch hierbei handelt es sich um ein nasschemisches Verfahren.

In einer weiteren Ausführungsform des erfindungsgemäßen Verfahrens wird zwischen den Schichten, die eine Mischung oder ein Mischoxid aus $Fe_2O_3$ und $TiO_2$ enthalten, zusätzlich mindestens eine weitere Schicht aufgebracht, die aus einem farblosen dielektrischen Material besteht, wobei das Material eine Brechzahl n von >1,8 aufweist. Wie vorab bereits beschrieben, handelt es sich hierbei vorzugsweise um eine $TiO_2$-Schicht, die entweder direkt auf die erste, eine Mischung oder ein Mischoxid aus $Fe_2O_3$ und $TiO_2$ enthaltende Schicht und/oder direkt auf die Zwischenschicht, die aus einem Material mit einer Brechzahl von ≤ 1,8 besteht, aufgebracht wird.

Das Aufbringen einer $TiO_2$-Schicht erfolgt dabei vorzugsweise analog zu dem in US 3,553,001 beschriebenen Verfahren. Dabei wird eine wässrige Titansalzlösung langsam zu einer Suspension des zu beschichtenden Pigmentes gegeben, die Suspension auf 50 bis 100 °C erhitzt und der pH-Wert im Bereich von 0,5 bis 5,0 durch gleichzeitige Zugabe einer Base, beispielsweise einer wässrigen Ammoniumhydroxidlösung oder einer wässrigen Alkalihydroxidlösung, nahezu konstant gehalten. Wenn die gewünschte $TiO_2$-Schichtdicke auf den Pigmentplättchen erreicht ist, wird die Zugabe der Titansalzlösung und der Base gestoppt. Da die Zugabe der Titansalzlösung so langsam erfolgt, dass eine quasi vollständige Abscheidung des Hydrolyseproduktes auf den Pigmentplättchen erfolgt, kommt es praktisch nicht zu Nebenfällungen. Das Verfahren ist als Titrationsverfahren bekannt.

Die Abscheidung einer zweiten, eine Mischung oder ein Mischoxid aus $Fe_2O_3$ und $TiO_2$ enthaltenden Schicht erfolgt analog zu der ersten Schicht dieser Art.

Vorzugsweise handelt es sich bei mindestens einer der Schichten, die eine Mischung oder ein Mischoxid aus $Fe_2O_3$ und $TiO_2$ enthalten, um eine Pseudobrookitschicht, die entweder aus Pseudobrookit besteht oder dieses zu einem Anteil von mindestens 80 %, vorzugsweise mindestens 90%, be-zogen auf das Gewicht der Schicht, enthält. Insbesondere weist das erfindungsgemäße goldene Interferenzigment zwei Schichten dieser Art auf.

Es soll nicht unerwähnt bleiben, dass die Beschichtung des vorab beschriebenen plättchenförmigen Substrates mit Interferenzschichten alternativ auch mittels eines Fliessbettreaktors durch Gasphasenabscheidung erfolgen kann. Dabei kann von den beispielsweise in EP 045 851 sowie EP 106 235 beschriebenen Technologien Gebrauch gemacht werden. Die vorab beschriebenen nasschemischen Verfahren sind jedoch bevorzugt.

Gegenstand der vorliegenden Erfindung ist auch die Verwendung der genannten goldenen Interferenzpigmente in Farben, Lacken, Druckfarben, Kunststoffen, Gläsern, Papier, Keramik, kosmetischen Formulierungen, zur Lasermarkierung von Kunststoffen oder Papier sowie zur Herstellung von Pigmentpräparationen und Trockenpräparaten. Dabei werden unter Pigmentpräparationen und Trockenpräparaten Pigmentanteigungen in Wasser und/oder organischen Lösemitteln, ggf. unter Zusatz von Bindemitteln und Hilfsstoffen, oder lösemittelarme oder-freie Präparate in Form von Granulaten, Pearlets, Chips, Pellets, Briketts, Würstchen, etc., verstanden. Insbesondere die letztgenannten Trockenpräparate werden vorzugsweise in Druckanwendungen eingesetzt, weil sie ein staubfreies Arbeiten ermöglichen.

Prinzipiell sind die goldenen Interferenzpigmente gemäß der vorliegenden Erfindung damit in allen gängigen Anwendungen einsetzbar, in denen gewöhnlich Effektpigmente, insbesondere Interferenzpigmente, verwendet werden können.

Sie können dort als alleinige Farbmittel eingesetzt oder aber auch in Abmischung mit anorganischen oder organischen Farbstoffen oder Pigmenten, beispielsweise Weiß-, Bunt- oder Schwarzpigmenten, mit LCPs (Liquid Crystal Pigments) und/oder mit anderen üblichen Effektpigmenten auf der Basis metallischer oder nichtmetallischer Substrate eingesetzt werden. Dabei sind alle denkbaren Mischungsverhältnisse möglich.

Selbstverständlich können die spezifischen Anwendungsmedien auch die allgemein üblichen Hilfs- und Zusatzstoffe sowie Bindemittel, Füllstoffe und/oder Lösemittel enthalten, ohne dass hier genauer darauf eingegangen werden muss.

Der Anteil der erfindungsgemäßen goldenen Interferenzpigmente im jeweiligen Anwendungsmedium ist abhängig vom konkreten Anwendungsfall und kann in einer breiten Konzentrationsspanne erfolgen.

Bei dem einsatzfähigen Anwendungsmedium handelt es sich oft um Beschichtungszusammensetzungen, die mittels üblicher Auftragsverfahren wie Drucken, Sprühen, Rakeln, Rollen, Streichen usw. auf die jeweiligen Untergründe, beispielsweise Bedruckstoffe, aufgebracht, getrocknet und wahlweise zusätzlich gehärtet bzw. vernetzt werden.

Besonders geeignet sind die vorliegenden goldenen Interferenzpigmente jedoch für Druckverfahren, und zwar für praktisch alle gängigen Druckverfahren. Besonders zu nennen sind hierbei das Tiefdruckverfahren, das Flexodruckverfahren, das Siebdruckverfahren, das Intagliodruckverfahren und das Offsetdruckverfahren.

Daher sind erfindungsgemäße Druckfarben, die das vorliegende goldene Interferenzpigment enthalten, insbesondere Tiefdruckfarben, Flexodruckfarben, Siebdruckfarben, Intagliodruckfarben oder Offsetdruckfarben. Diese können das erfindungsgemäße Interferenzpigment in der üblichen Pigmentierung, die in der Regel zwischen 1 und 35 Gew.%, ausnahmsweise bis zu 40 Gew.%, bezogen auf das Gewicht der Druckfarbe, beträgt, enthalten.

Alle anderen Druckfarbenbestandteile, wie Bindemittel, Lösemittel, Füllstoffe, Photoinitiatoren, Härter, Verlaufsverzögerer, Netzmittel, Trockenmittel, etc., um nur einige zu nennen, die fachüblich verwendet werden, können selbstverständlich ebenso in den üblicherweise gebräuchlichen Konzentrationen gleichzeitig mit dem erfindungsgemäßen goldenen Interferenzpigment in den jeweiligen Druckfarben enthalten sein. Bezüglich der einsetzbaren pigmentfreien Halb- und Fertigprodukte können die üblichen Druckfarbenvehikel der am Markt etablierten Hersteller verwendet werden.

Von besonderer Bedeutung ist die Anwendung der erfindungsgemäßen Pigmente in Druckverfahren, die aus verschiedenen Gründen nur mit sehr feinteiligen Pigmenten arbeiten können. Hier sind insbesondere das Offsetdruckverfahren und das Intagliodruckverfahren (Stahlstichdruckverfahren mit pastösen, hochviskosen Druckfarben) zu nennen.

Die erfindungsgemäßen Interferenzpigmente erfüllen hier die Anforderung an die Feinteiligkeit der einzusetzenden Pigmente, ohne dass Nachteile in Bezug auf den Glanz, die Farbsättigung oder den gewünschten warmen Goldton der resultierenden Drucke hingenommen werden müssen. Ihr Einsatz kann in den üblichen Pigmentkonzentrationen für die genannten Verfahren und in Kombination mit den anderen gängigen Inhaltsstoffen von geeigneten Druckfarbenvehikeln bzw. Druckfarben erfolgen. Außerdem sind die erfindungsgemäßen Interferenzpigmente in der Lage, sich auf dem Bedruckstoff trotz ihrer Feinteiligkeit gut auszurichten sowie im Druckbild eine hohe Farbsättigung im Rot-Gold-Bereich aufzuweisen, auch wenn die erzeugte Druckschicht, wie beim Offsetdruckverfahren, nur bis zu maximal 3 Mikrometer dünn ist.

Die erfindungsgemäßen Interferenzpigmente verstopfen die Druckformen (z. B. Gravuren in Druckplatten und -zylindern) und die Zuleitungen nicht und sorgen daher für saubere Druckbilder sowie ein gutes Fortdruckverhalten in der Massenherstellung von Druckerzeugnissen. Außerdem sind sie chemisch und mechanisch sehr stabil, so dass die beispielsweise im Intagliodruckverfahren eingesetzten stark basischen Wischlösungen keine negativen Auswirkungen auf die optischen Charakteristika der erfindungsgemäßen Pigmente haben.

Die goldenen Interferenzpigmente gemäß der vorliegenden Erfindung sind daher in besonderer Weise für die Anwendung in den meisten gebräuchlichen, kostengünstigen Druck- und Beschichtungsverfahren geeignet und dienen zur Erzeugung von Golddrucken und Golddekoren sowohl im Verpackungsbereich wie auch im dekorativen Bereich und insbesondere auch im Sicherheitsbereich, wo sie beispielsweise zur Herstellung von Banknoten und anderen Wertpapieren unter Anwendung der dort üblichen, speziellen Beschichtungsverfahren, beispielsweise dem Intagliodruckverfahren, vorteilhaft eingesetzt werden können. Bei dem technisch in großem Umfang für das Bedrucken von Textilien und Papier verwendeten Siebdruckverfahren ist die Verwendung engmaschigerer Siebe und damit das Drucken feinerer Linien in einem satten Rot-Gold-Farbton möglich.

Die vorliegende Erfindung soll anhand der folgenden Beispiele näher erläutert, jedoch nicht auf diese beschränkt werden.

Beispiel 1:

200 g Aluminiumdioxid-Flakes mit grüner Interferenzfarbe, die eine Teilchengrößenverteilung $d_{50}$=18-19,5 $\mu$m und $d_{95}$=37-39 $\mu$m (bestimmt mit Malvern Mastersizer 2000) und eine geometrische Dicke von ca. 220 nm (bestimmt über SEM) aufweisen, werden in 2 l entmineralisiertem Wasser suspendiert und die Suspension auf eine Temperatur von 75°C aufgeheizt. Nach Erreichen dieser Temperatur wird unter Rühren eine Lösung aus 248,0 g $FeCl_3 \times 6H_2O$, 87,0 g $TiCl_4$ und 10,4 g $AlCl_3 \times 6 H_2O$ in 291,2 g entmineralisiertem Wasser langsam zudosiert. Der pH-Wert der Suspension wird mit NaOH-Lösung (32%) konstant bei 2,6 gehalten. Nach Zugabe der Metallsalzlösungen wird etwa 15 Minuten nachgerührt. Anschließend wird der pH-Wert mit NaOH-Lösung (32%) auf pH 7,5 angehoben und bei diesem pH-Wert 592,6 g Natronwaserglaslösung (13,5% $SiO_2$) langsam zugegeben. Danach wird der pH-Wert mit Salzsäure (10% HCl) auf 2,0 abgesenkt und 15 Minuten nachgerührt. Anschließend werden 192 ml $TiCl_4$-Lösung (370 g $TiCl_4$/l) zudosiert. Dabei wird der pH-Wert mit NaOH-Lösung (32%) konstant gehalten. Anschließend wird der pH-Wert mit NaOH-Lösung (32%) auf 2,6 erhöht und bei diesem Wert 264,8 g $FeCl_3 \times 6 H_2O$, 92,6 g $TiCl_4$ und 11,0 g $AlCl_3 \times 6 H_2O$ in 133,6 g entmineralisiertem Wasser langsam zudosiert. Der pH-Wert wird mit NaOH-Lösung (32%) konstant gehalten. Anschließend wird 15 Minuten nachgerührt, der pH-Wert auf pH 5,0 angehoben (NaOH-Lösung, 32%) und weitere 15 Minuten nachgerührt. Das Pigment wird filtert, mit entmineralisiertem Wasser gewaschen und bei 110°C getrocknet. Anschließend wird es bei 850°C 30 Minuten lang geglüht.

Es wird ein goldfarbenes Glanzpigment mit rotgoldener Interferenz- und Körperfarbe, starkem Glanz sowie einem sehr guten Deckvermögen erhalten.

Vergleichsbeispiel 1:

100 g Glimmer-Flakes mit einer Teilchengröße von 10-60 $\mu$m werden in 2 l entmineralisiertem Wasser suspendiert und die Suspension auf eine Temperatur von 75°C aufgeheizt. Nach Erreichen dieser Temperatur wird unter Rühren eine Lösung aus 130,5 g $FeCl_3 \times 6H_2O$, 46,5 g $TiCl_4$ und 11,6 g $AlCl_3 \times 6 H_2O$ in 84,3 g entmineralisiertem Wasser langsam zudosiert. Der pH-Wert der Suspension wird mit NaOH-Lösung (32%) konstant bei 2,6 gehalten. Nach Zugabe der Metallsalzlösungen wird etwa 15 Minuten nachgerührt. Anschließend wird der pH-Wert mit NaOH-Lösung (32%) auf pH 7,5 angehoben und bei diesem pH-Wert 431 g Natronwasserglaslösung (13,5% $SiO_2$) langsam zugegeben. Danach wird der pH-Wert mit Salzsäure (10% HCl) auf 2,0 abgesenkt und 15 Minuten nachgerührt. Anschließend werden 393 g $TiCl_4$-Lösung (370 g $TiCl_4$/l) zudosiert. Dabei wird der pH-Wert mit NaOH-Lösung (32%) konstant gehalten. Anschließend wird der pH-Wert mit NaOH-Lösung (32%) auf 2,6 erhöht und bei diesem Wert 48,6 g $FeCl_3 \times 6 H_2O$, 18,6 g $TiCl_4$ und 4,0 g $AlCl_3 \times 6 H_2O$ in 31,4 g entmineralisiertem Wasser langsam zudosiert. Der pH-Wert wird mit NaOH-Lösung (32%) konstant gehalten. Anschließend wird 15 Minuten nachgerührt, der pH-Wert auf pH 5,0 angehoben (NaOH-Lösung, 32%) und weitere 15 Minuten nachgerührt. Das Pigment wird filtert, mit entmineralisiertem Wasser gewaschen und bei 110°C getrocknet. Anschließend wird es bei 850°C 30 Minuten lang geglüht.

Es wird ein goldfarbenes Glanzpigment mit goldener Interferenzfarbe, einem starken Glanz, extrem hoher Helligkeit sowie einem guten Deckvermögen erhalten.

Von den Goldpigmenten gemäß Beispiel 1 und Vergleichsbeispiel 1 werden jeweils Schwarz-Weiß-Lackkarten angefertigt. Die entsprechenden CIEL,a,b-Werte werden mit einem ETA-Device (STEAG-ETA Optic GmbH Inc.) ermittelt.

| Probe | 75°/95° Schwarzer Hintergrund | | Chroma *C | Farbwinkel *h | Deckvermögen |
|---|---|---|---|---|---|
| | L | ab | | | |
| Bsp. 1 | 117,8 | 19,8 | 96,4 | 98,4 | 78,4 | 35,8 |
| Vgl.Bsp. | 158,9 | 4,3 | 104,0 | 104,1 | 87,6 | 30,8 |

Die vorab gezeigten Werte zeigen, dass das erfindungsgemäße Pigment gemäß Beispiel 1 eine hohe Helligkeit, einen sehr guten Chromawert (Buntheit) sowie ein ausgesprochen hohes Deckvermögen aufweist. Darüber hinaus zeigt es bei einem Farbtonwinkel von etwa 78° einen deutlich rötlicheren Farbton als das Vergleichspigment bei einem Farbtonwinkel von etwa 87°. Dagegen weist das Vergleichspigment eine so große Helligkeit auf, dass mit bloßem Auge ein gleißender Gelbton, nicht jedoch ein warmer Rotgold-Ton wahrgenommen wird. Das Deckvermögen des Vergleichspigmentes bleibt dagegen deutlich hinter dem des erfindungsgemäßen Pigmentes gemäß Beispiel 1 zurück. Darüber hinaus ist das Pigment gemäß Vergleichsbeispiel 1 wegen der vergleichsweise großen Partikelgröße nicht für Anwendungen geeignet, die sehr feinteilige Pigmente erfordern.

(Im CIELab-System ist die Sättigung einer Farbe nur unzureichend beschrieben und wird oft mit der Buntheit gleichgesetzt. Nach Eva Lübbe, Sättigung im CIELAB-Farbsystem und LSh-Farbsystem, Books on Demand GmbH, Norderstedt, 3. Auflage 2011, S. 47, wird die Sättigung einer Farbe aber besser durch das Verhältnis der Buntheit der Farbe zum Gesamtfarbeindruck charakterisiert. Danach wird die Sättigung S wie folgt berechnet:

$$S = \frac{C^*_{ab}}{\sqrt{L^{*2} - C^{*2}_{ab}}} \cdot 100\%$$

wobei S die neu berechnete Sättigung, L* der Helligkeitswert nach CIELAB und C*$_{ab}$ die Buntheit nach CIELAB darstellen.

Werden nach der angegebenen Formel die Sättigung der Proben gemäß Beispiel 1 sowie des Vergleichsbeispiels berechnet, ergibt sich für das Beispiel 1 ein Wert für die Sättigung von etwa 64%, während die Sättigung im Vergleichsbeispiel lediglich etwa 55% beträgt. Diese Werte entsprechen der visuellen Wahrnehmung, die für die Lackkarte des Beispiels 1 eine deutlich höhere Farbsättigung ergibt als für die des Vergleichsbeispiels.)

Anwendungsbeispiele:

1. Intagliodruckfarbe:

| Goldpigment gemäß Beispiel 1 | 15 Gew.% |
| Intaglio Varnish Flop 670179 | 85 Gew.% |

(Fa. Gleitsmann Security Inks)

Das Goldpigment wird in das Bindemittelsystem schonend eingearbeitet und mit einer auf 40°C bis 70°C erwärmten Stahlstichdruckplatte auf Papier gedruckt. Es wird ein erhabenes Muster aus feinen Linien mit einem warmen, rotgoldenen Farbton in guter Sättigung erhalten.

2. Offsetfarbe:

a)

| Goldpigment gemäß Beispiel 1 | 15 Gew.% |
| OF Drucklack 96147 | 85 Gew.% |

(Fa. Jaenecke und Schneemann Druckfarbe GmbH)
b)

| Goldpigment gemäß Beispiel 1 | 30 Gew.% |
| OF Drucklack 96147 | 70 Gew.% |

(Fa. Jaenecke und Schneemann Druckfarbe GmbH)

Das Goldpigment wird jeweils schonend in das Druckfarbenvehikel eingearbeitet und die erhaltenen Druckfarbe verdruckt. In beiden Fällen wird ein gut sichtbares, auffällig rot-goldenes Muster erhalten, dessen wahrnehmbare Farbsättigung bei dem Druckergebnis gemäß 2b deutlich stärker ausfällt als bei dem Druckergebnis gemäß Beispiel 2a.

3. Siebdruckfarbe:

Das Goldpigment gemäß Beispiel 1 wird in Anteilen zu je 10 Gew.% bzw. 15 Gew.% in jeweils 90 Gew.% bzw. 85 Gew.% Siebdruckbinder (AquaJet FGLM 093 oder MZ Lack 093, Fa. Pröll KG, oder UV-wässrig 672048, Fa. Gleitsmann Security Inks) schonend eingebracht. Das Verdrucken erfolgt mit handelsübliche Sieben (61-64 bzw. 77-55).

Die erhaltenen Druckfarben lassen sich mit jedem der Siebe erfolgreich verdrucken, ohne die Siebe zu verstopfen. In jeder Konzentration und mit jedem der aufgeführten Bindemittel werden satte, rot-goldene, stark deckende Druckbilder mit hohem Glanz erhalten.

4. Tiefdruckfarbe/Flexodruckfarbe:

Das Goldpigment gemäß Beispiel 1 wird in Anteilen zu je 15 Gew.% bzw. 25 Gew.% in jeweils 85 Gew.% bzw. 75 Gew.% Tief-/Flexodruckbinder (NC TOB OPV-00, Fa. Siegwerk, oder Haptobond CT 105, Fa. Hartmann Druckfarben GmbH/Sun Chemical) schonend eingerührt. Die Viskosität der Druckfarbe wird mit geringen Mengen an Lösemittel eingestellt.

Die erhaltenen Druckbilder zeigen einen satten Rot-Gold-Farbton sowie einen hohen Glanz.

**Patentansprüche**

1. Goldenes Interferenzpigment, welches ein plättchenförmiges Substrat und mindestens eine auf dem Substrat befindliche Schicht umfasst, **dadurch gekennzeichnet, dass** es sich bei dem plättchenförmigen Substrat um ein synthetisch erzeugtes transparentes Substrat handelt, welches als solches eine grüne Eigeninterferenzfarbe aufweist, wobei das Substrat aus $Al_2O_3$ oder aus $Al_2O_3$ mit einem Gehalt von bis zu 5 Gew.% $TiO_2$, bezogen auf das Gewicht des Substrates, besteht oder $Al_2O_3$ mit einem Anteil von mindestens 90 Gew.%, bezogen auf das Gewicht des Substrates, enthält und eine geometrische Dicke im Bereich von 180 bis 250 nm oder im Bereich von 350 bis 450 nm aufweist, oder aus $ZrO_2$ besteht oder $ZrO_2$ mit einem Anteil von mindestens 90 Gew.%, bezogen auf das Gewicht des Substrates, enthält und eine geometrische Schichtdicke im Bereich von 140 bis 210 nm oder im Bereich von 260 bis 400 nm aufweist, oder aus $TiO_2$ besteht oder $TiO_2$ mit einem Anteil von mindestens 90 Gew.%, bezogen auf das Gewicht des Substrates, enthält und eine geometrische Schichtdicke im Bereich von 110 bis 170 nm oder im Bereich von 240 bis 310 nm aufweist, oder wobei es sich um ein Glasplättchen mit einem $SiO_2$-Anteil von maximal 70 Gew.% handelt, welches eine geometrische Schichtdicke im Bereich von 230 bis 300 nm oder von 400 bis 470 nm aufweist, und wobei sich auf dem Substrat mindestens eine Schicht befindet, die eine Mischung oder ein Mischoxid aus $Fe_2O_3$ und $TiO_2$ enthält und das Substrat eine Teilchengröße im Bereich von 5-40 µm und einen $d_{95}$-Wert im Bereich von 35 µm bis < 40 µm aufweist.

2. Interferenzpigment gemäß Anspruch 1, **dadurch gekennzeichnet, dass** das Pigment zwei Schichten aufweist, die eine Mischung oder ein Mischoxid aus $Fe_2O_3$ und $TiO_2$ enthalten.

3. Interferenzpigment gemäß Anspruch 2, **dadurch gekennzeichnet, dass** zwischen den Schichten, die eine Mischung oder ein Mischoxid aus $Fe_2O_3$ und $TiO_2$ enthalten, mindestens eine weitere Schicht aus einem farblosen dielektrischen Material enthalten ist, wobei das Material eine Brechzahl n von ≤ 1,8 aufweist.

4. Interferenzpigment gemäß Anspruch 3, **dadurch gekennzeichnet, dass** zwischen den Schichten, die eine Mischung oder ein Mischoxid aus $Fe_2O_3$ und $TiO_2$ enthalten, zusätzlich eine weitere Schicht aus einem farblosen dielektrischen Material enthalten ist, wobei das Material eine Brechzahl n von >1,8 aufweist.

5. Interferenzpigment gemäß einem oder mehreren der Ansprüche 1 bis 4, **dadurch gekennzeichnet, dass** die Schicht, die eine Mischung oder ein Mischoxid aus $Fe_2O_3$ und $TiO_2$ enthält, eine geometrische Schichtdicke im Bereich von 30 nm bis 180 nm aufweist.

6. Interferenzpigment gemäß einem oder mehreren der Ansprüche 1 bis 5, **dadurch gekennzeichnet, dass** es sich bei mindestens einer der Schichten, die eine Mischung oder ein Mischoxid aus $Fe_2O_3$ und $TiO_2$ enthalten, um eine Pseudobrookitschicht handelt.

7. Verfahren zur Herstellung eines goldenen Interferenzpigmentes gemäß einem oder mehreren der Ansprüche 1 bis 6, **dadurch gekennzeichnet, dass** ein synthetisch erzeugtes transparentes Substrat, welches eine grüne Eigeninterferenzfarbe aufweist, wobei das Substrat aus $Al_2O_3$ oder aus $Al_2O_3$ mit einem Gehalt von bis zu 5 Gew.% $TiO_2$, bezogen auf das Gewicht des Substrates, besteht oder $Al_2O_3$ mit einem Anteil von mindestens 90 Gew.%, bezogen auf das Gewicht des Substrates, enthält und eine geometrische Dicke im Bereich von 180 bis 250 nm oder im Bereich von 350 bis 450 nm aufweist, oder aus $ZrO_2$ besteht oder $ZrO_2$ mit einem Anteil von mindestens 90 Gew.%, bezogen auf das Gewicht des Substrates, enthält und eine geometrische Schichtdicke im Bereich von 140 bis 210 nm oder im Bereich von 260 bis 400 nm aufweist, oder aus $TiO_2$ besteht oder $TiO_2$ mit einem Anteil von mindestens 90 Gew.%, bezogen auf das Gewicht des Substrates, enthält und eine geometrische Schichtdicke im Bereich von 110 bis 170 nm oder im Bereich von 240 bis 310 nm aufweist, oder wobei es sich um ein Glasplättchen mit einem $SiO_2$-Anteil von maximal 70 Gew.% handelt, welches eine geometrische Schichtdicke im Bereich von 230 bis 300 nm oder von 400 bis 470 nm aufweist, und wobei das Substrat eine Teilchengröße im Bereich von 5-40 µm und

einen $d_{95}$-Wert im Bereich von 35 $\mu$m bis < 40 $\mu$m aufweist, mit mindestens einer Schicht belegt wird, die eine Mischung oder ein Mischoxid aus $Fe_2O_3$ und $TiO_2$ enthält.

8. Verfahren gemäß Anspruch 7, **dadurch gekennzeichnet, dass** die Belegung in einer wässrigen Dispersion mittels eines nasschemischen Verfahrens durch hydrolytische Zersetzung von anorganischen Metallsalzen erfolgt.

9. Verfahren gemäß Anspruch 7 oder 8, **dadurch gekennzeichnet, dass** zwei Schichten aufgebracht werden, die eine Mischung oder ein Mischoxid aus $Fe_2O_3$ und $TiO_2$ enthalten, wobei zwischen diesen Schichten mindestens eine weitere Schicht aus einem farblosen dielektrischen Material aufgebracht wird, wobei das Material für die weitere Schicht eine Brechzahl n von <1,8 aufweist.

10. Verfahren gemäß Anspruch 9, **dadurch gekennzeichnet, dass** zwischen den Schichten, die eine Mischung oder ein Mischoxid aus $Fe_2O_3$ und $TiO_2$ enthalten, zusätzlich mindestens eine weitere Schicht aufgebracht wird, die aus einem farblosen dielektrischen Material besteht, wobei das Material eine Brechzahl n von >1,8 aufweist.

11. Verfahren gemäß einem oder mehreren der Ansprüche 7 bis 10, **dadurch gekennzeichnet, dass** mindestens eine der Schichten, die eine Mischung oder ein Mischoxid aus $Fe_2O_3$ und $TiO_2$ enthalten, eine Pseudobrookitschicht ist.

12. Verwendung von Interferenzpigmenten gemäß einem oder mehreren der Ansprüche 1 bis 6 in Farben, Lacken, Druckfarben, Kunststoffen, Gläsern, Papier, Keramik, kosmetischen Formulierungen, zur Lasermarkierung von Kunststoffen oder Papier sowie zur Herstellung von Pigmentpräparationen und Trockenpräparaten.

13. Verwendung gemäß Anspruch 12, **dadurch gekennzeichnet, dass** es sich bei der Druckfarbe um eine Tiefdruckfarbe, eine Flexodruckfarbe, eine Siebdruckfarbe, eine Intagliodruckfarbe oder um eine Offsetdruckfarbe handelt.

14. Tiefdruckfarbe, Flexodruckfarbe, Siebdruckfarbe, Intagliodruckfarbe oder Offsetdruckfarbe, enthaltend Interferenzpigmente gemäß einem oder mehreren der Ansprüche 1 bis 6.

**Claims**

1. Golden interference pigment which comprises a flake-form substrate and at least one layer located on the substrate, **characterised in that** the flake-form substrate is a synthetically produced transparent substrate which has per se a green inherent interference colour, where the substrate consists of $Al_2O_3$ or of $Al_2O_3$ with a content of up to 5% by weight of $TiO_2$, based on the weight of the substrate, or comprises $Al_2O_3$ with a proportion of at least 90% by weight, based on the weight of the substrate, and has a geometrical thickness in the range from 180 to 250 nm or in the range from 350 to 450 nm, or consists of $ZrO_2$ or comprises $ZrO_2$ with a proportion of at least 90% by weight, based on the weight of the substrate, and has a geometrical layer thickness in the range from 140 to 210 nm or in the range from 260 to 400 nm, or consists of $TiO_2$ or comprises $TiO_2$ with a proportion of at least 90% by weight, based on the weight of the substrate, and has a geometrical layer thickness in the range from 110 to 170 nm or in the range from 240 to 310 nm, or where it is a glass flake having an $SiO_2$ proportion of at most 70% by weight which has a geometrical layer thickness in the range from 230 to 300 nm or from 400 to 470 nm, and where at least one layer which comprises a mixture or mixed oxide of $Fe_2O_3$ and $TiO_2$ is located on the substrate and the substrate has a particle size in the range 5-40 $\mu$m and a $d_{95}$ value in the range from 35 $\mu$m to < 40 $\mu$m.

2. Interference pigment according to Claim 1, **characterised in that** the pigment has two layers which comprise a mixture or mixed oxide of $Fe_2O_3$ and $TiO_2$.

3. Interference pigment according to Claim 2, **characterised in that** at least one further layer comprising a colourless dielectric material, where the material has a refractive index n of $\leq 1.8$, is present between the layers that comprise a mixture or mixed oxide of $Fe_2O_3$ and $TiO_2$.

4. Interference pigment according to Claim 3, **characterised in that** a further layer comprising a colourless dielectric material, where the material has a refractive index n of >1.8, is additionally present between the layers that comprise a mixture or mixed oxide of $Fe_2O_3$ and $TiO_2$.

5. Interference pigment according to one or more of Claims 1 to 4, **characterised in that** the layer that comprises a mixture or mixed oxide of $Fe_2O_3$ and $TiO_2$ has a geometrical layer thickness in the range from 30 nm to 180 nm.

6. Interference pigment according to one or more of Claims 1 to 5, **characterised in that** at least one of the layers that comprise a mixture or mixed oxide of $Fe_2O_3$ and $TiO_2$ is a pseudobrookite layer.

7. Process for the preparation of a golden interference pigment according to one or more of Claims 1 to 6, **characterised in that** a synthetically produced transparent substrate which has a green inherent interference colour, where the substrate consists of $Al_2O_3$ or of $Al_2O_3$ with a content of up to 5% by weight of $TiO_2$, based on the weight of the substrate, or comprises $Al_2O_3$ with a proportion of at least 90% by weight, based on the weight of the substrate, and has a geometrical thickness in the range from 180 to 250 nm or in the range from 350 to 450 nm, or consists of $ZrO_2$ or comprises $ZrO_2$ with a proportion of at least 90% by weight, based on the weight of the substrate, and has a geometrical layer thickness in the range from 140 to 210 nm or in the range from 260 to 400 nm, or consists of $TiO_2$ or comprises $TiO_2$ with a proportion of at least 90% by weight, based on the weight of the substrate, and has a geometrical layer thickness in the range from 110 to 170 nm or in the range from 240 to 310 nm, or where it is a glass flake having an $SiO_2$ proportion of at most 70% by weight which has a geometrical layer thickness in the range from 230 to 300 nm or from 400 to 470 nm, and where the substrate has a particle size in the range 5-40 $\mu$m and a $d_{95}$ value in the range from 35 $\mu$m to < 40 $\mu$m, is coated with at least one layer which comprises a mixture or mixed oxide of $Fe_2O_3$ and $TiO_2$.

8. Process according to Claim 7, **characterised in that** the coating is carried out in an aqueous dispersion by means of a wet-chemical process by hydrolytic decomposition of inorganic metal salts.

9. Process according to Claim 7 or 8, **characterised in that** two layers which comprise a mixture or mixed oxide of $Fe_2O_3$ and $TiO_2$ are applied, where at least one further layer comprising a colourless dielectric material is applied between these layers, where the material for the further layer has a refractive index n of < 1.8.

10. Process according to Claim 9, **characterised in that** at least one further layer which consists of a colourless dielectric material, where the material has a refractive index n of > 1.8, is additionally applied between the layers that comprise a mixture or mixed oxide of $Fe_2O_3$ and $TiO_2$.

11. Process according to one or more of Claims 7 to 10, **characterised in that** at least one of the layers that comprise a mixture or mixed oxide of $Fe_2O_3$ and $TiO_2$ is a pseudobrookite layer.

12. Use of interference pigments according to one or more of Claims 1 to 6 in paints, coatings, printing inks, plastics, glasses, paper, ceramic, cosmetic formulations, for the laser marking of plastics or paper and for the preparation of pigment preparations and dry preparations.

13. Use according to Claim 12, **characterised in that** the printing ink is a gravure printing ink, a flexographic printing ink, a screen printing ink, an intaglio printing ink or an offset printing ink.

14. Gravure printing ink, flexographic printing ink, screen printing ink, intaglio printing ink or offset printing ink comprising interference pigments according to one or more of Claims 1 to 6.

**Revendications**

1. Pigment d'interférence doré qui comprend un substrat en forme de paillettes et au moins une couche située sur le substrat, **caractérisé en ce que** le substrat en forme de paillettes est un substrat transparent produit synthétiquement qui possède en soi une couleur d'interférence inhérente verte, où le substrat est constitué d'$Al_2O_3$ ou d'$Al_2O_3$ avec une teneur allant jusqu'à 5% en poids de $TiO_2$, sur la base du poids du substrat, ou comprend de l'$Al_2O_3$ selon une proportion d'au moins 90% en poids, sur la base du poids du substrat, et présente une épaisseur géométrique dans la plage allant de 180 à 250 nm ou dans la plage allant de 350 à 450 nm, ou est constitué de $ZrO_2$ ou comprend du $ZrO_2$ selon une proportion d'au moins 90% en poids, sur la base du poids du substrat, et présente une épaisseur de couche géométrique dans la plage allant de 140 à 210 nm ou dans la plage allant de 260 à 400 nm, ou est constitué de $TiO_2$ ou comprend du $TiO_2$ selon une proportion d'au moins 90% en poids, sur la base du poids du substrat, et présente une épaisseur de couche géométrique dans la plage allant de 110 à 170 nm ou dans la plage allant de 240 à 310 nm, ou où il s'agit d'une paillette de verre ayant une proportion de $SiO_2$ d'au plus 70% en poids qui présente une épaisseur de couche géométrique dans la plage allant de 230 à 300 nm ou de 400 à 470 nm, et où au moins une couche qui comprend un mélange ou un oxyde mixte de $Fe_2O_3$ et de $TiO_2$ est située sur le substrat et le substrat présente une taille de particules dans la plage de 5-40 $\mu$m et une valeur de $d_{95}$ dans la plage allant

de 35 $\mu$m à < 40 $\mu$m.

2. Pigment d'interférence selon la revendication 1, **caractérisé en ce que** le pigment possède deux couches qui comprennent un mélange ou un oxyde mixte de $Fe_2O_3$ et de $TiO_2$.

3. Pigment d'interférence selon la revendication 2, **caractérisé en ce qu'**au moins une couche supplémentaire comprenant un matériau diélectrique incolore, où le matériau présente un indice de réfraction n $\leq$ 1,8, est présente entre les couches qui comprennent un mélange ou un oxyde mixte de $Fe_2O_3$ et de $TiO_2$.

4. Pigment d'interférence selon la revendication 3, **caractérisé en ce qu'**une couche supplémentaire comprenant un matériau diélectrique incolore, où le matériau présente un indice de réfraction n >1,8, est en outre présente entre les couches qui comprennent un mélange ou un oxyde mixte de $Fe_2O_3$ et de $TiO_2$.

5. Pigment d'interférence selon l'une ou plusieurs parmi les revendications 1 à 4, **caractérisé en ce que** la couche qui comprend un mélange ou un oxyde mixte de $Fe_2O_3$ et de $TiO_2$ présente une épaisseur de couche géométrique dans la plage allant de 30 nm à 180 nm.

6. Pigment d'interférence selon l'une ou plusieurs parmi les revendications 1 à 5, **caractérisé en ce qu'**au moins l'une des couches qui comprend un mélange ou un oxyde mixte de $Fe_2O_3$ et de $TiO_2$ est une couche de pseudobrookite.

7. Procédé de préparation d'un pigment d'interférence doré selon l'une ou plusieurs parmi les revendications 1 à 6, **caractérisé en ce qu'**un substrat transparent produit synthétiquement qui possède une couleur d'interférence inhérente verte, où le substrat est constitué d'$Al_2O_3$ ou d'$Al_2O_3$ avec une teneur allant jusqu'à 5% en poids de $TiO_2$, sur la base du poids du substrat, ou comprend de l'$Al_2O_3$ selon une proportion d'au moins 90% en poids, sur la base du poids du substrat, et présente une épaisseur géométrique dans la plage allant de 180 à 250 nm ou dans la plage allant de 350 à 450 nm, ou est constitué de $ZrO_2$ ou comprend du $ZrO_2$ selon une proportion d'au moins 90% en poids, sur la base du poids du substrat, et présente une épaisseur de couche géométrique dans la plage allant de 140 à 210 nm ou dans la plage allant de 260 à 400 nm, ou est constitué de $TiO_2$ ou comprend du $TiO_2$ selon une proportion d'au moins 90% en poids, sur la base du poids du substrat, et présente une épaisseur de couche géométrique dans la plage allant de 110 à 170 nm ou dans la plage allant de 240 à 310 nm, ou où il s'agit d'une paillette de verre ayant une proportion de $SiO_2$ d'au plus 70% en poids qui présente une épaisseur de couche géométrique dans la plage allant de 230 à 300 nm ou de 400 à 470 nm, et où le substrat présente une taille de particules dans la plage de 5-40 $\mu$m et une valeur de $d_{95}$ dans la plage allant de 35 $\mu$m à < 40 $\mu$m, est revêtu par au moins une couche qui comprend un mélange ou un oxyde mixte de $Fe_2O_3$ et de $TiO_2$.

8. Procédé selon la revendication 7, **caractérisé en ce que** le revêtement est réalisé dans une dispersion aqueuse au moyen d'un procédé de chimie par voie humide par décomposition hydrolytique de sels métalliques inorganiques.

9. Procédé selon la revendication 7 ou 8, **caractérisé en ce que** deux couches qui comprennent un mélange ou un oxyde mixte de $Fe_2O_3$ et de $TiO_2$ sont appliquées, où au moins une couche supplémentaire comprenant un matériau diélectrique incolore est appliquée entre ces couches, où le matériau de la couche supplémentaire présente un indice de réfraction n < 1,8.

10. Procédé selon la revendication 9, **caractérisé en ce qu'**au moins une couche supplémentaire qui est constitué d'un matériau diélectrique incolore, où le matériau présente un indice de réfraction n > 1,8, est appliquée en outre entre les couches qui comprennent un mélange ou un oxyde mixte de $Fe_2O_3$ et de $TiO_2$.

11. Procédé selon l'une ou plusieurs parmi les revendications 7 à 10, **caractérisé en ce qu'**au moins l'une des couches qui comprennent un mélange ou un oxyde mixte de $Fe_2O_3$ et de $TiO_2$ est une couche de pseudobrookite.

12. Utilisation de pigments d'interférence selon l'une ou plusieurs parmi les revendications 1 à 6, dans les peintures, les revêtements, les encres d'impression, les matières plastiques, les verres, les papiers, les céramiques, les formulations cosmétiques, pour le marquage au laser de matières plastiques ou de papiers et pour la préparation de préparations de pigments et de préparations sèches.

13. Utilisation selon la revendication 12, **caractérisée en ce que** l'encre d'impression est une encre pour héliogravure, une encre pour impression flexographique, une encre pour impression sérigraphique, une encre pour impression sur caoutchouc ou une encre pour impression offset.

**14.** Encre pour héliogravure, encre pour impression flexographique, encre pour impression sérigraphique, encre pour impression sur caoutchouc ou encre pour impression offset, comprenant des pigments d'interférence selon l'une ou plusieurs parmi les revendications 1 à 6.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19618569 A1
- DE 19915153 A1
- DE 19951871 A1
- DE 19951869 A1
- EP 1980594 A1
- EP 0307747 A1
- EP 0763573 A2
- EP 271767 B1
- DE 2522572 C2
- US 6626989 B
- DE 2215191
- DE 3151354
- DE 3235017
- DE 3334598
- EP 0090259 A
- EP 634459 A
- WO 9632446 A
- WO 9957204 A
- WO 0192425 A
- EP 763573 A2

- JP 57111239 A
- JP 4039362 A
- WO 9308237 A
- DE 1467468
- DE 1959988
- DE 2009566
- DE 2214
- DE 545
- DE 2244298
- DE 2313331
- DE 2522572
- DE 3137808
- DE 3137809
- DE 3151343
- DE 3151355
- DE 3211602
- US 3553001 A
- EP 045851 A
- EP 106235 A